(19) Europäisches Patentamt
European Patent Office
Office européen des brevets

(11) Veröffentlichungsnummer: **0 253 378**
**A2**

(12) **EUROPÄISCHE PATENTANMELDUNG**

(21) Anmeldenummer: **87110222.4**

(22) Anmeldetag: **15.07.87**

(51) Int. Cl.⁴: **C 07 D 493/22**, C 07 F 7/18, A 01 N 43/90 // (C07D493/22, 313:00, 311:00, 311:00, 307:00)

(30) Priorität: **18.07.86 CH 2877/86**

(43) Veröffentlichungstag der Anmeldung: **20.01.88 Patentblatt 88/3**

(84) Benannte Vertragsstaaten: **AT BE CH DE ES FR GB GR IT LI LU NL SE**

(71) Anmelder: **CIBA-GEIGY AG, Klybeckstrasse 141, CH-4002 Basel (CH)**

(72) Erfinder: **Gubler, Kurt, Dr., Eulenweg 4, CH-4125 Riehen (CH)**
Erfinder: **Tsukamoto, Yoshihisa, 1-14-9, Kusatsu, Kusatsu-shi Shiga-ken (JP)**
Erfinder: **Sato, Kazuo, Dr., 5030 Centre Avenue 664, Pittsburgh, PA 15213 (US)**
Erfinder: **Yanai, Toshiaki, 1120-2, Ojinohara Yasu-cho, Yasu-gun Shiga-ken (JP)**

(74) Vertreter: **Zumstein, Fritz, Dr. et al, Bräuhausstrasse 4, D-8000 München 2 (DE)**

(54) **13Beta-Alkyl-derivate von S541-Antibiotika zur Bekämpfung von Parasiten an Nutztieren und Pflanzen.**

(57) Es werden parasitizid und insektizid hochaktive Wirkstoffe der Formel I

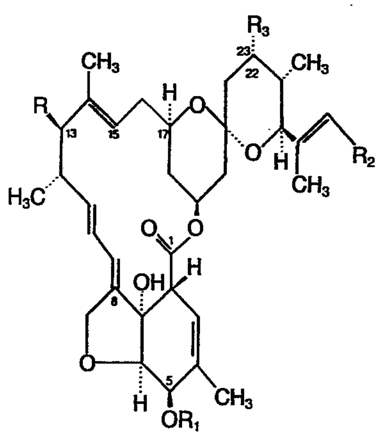

(I),

worin
R für $C_1$-$C_{10}$-Alkyl steht;
$R_1$ Wasserstoff, eine Methyl-, Acyl- oder eine Silylgruppe repräsentiert; $R_2$ für Methyl, Ethyl oder Isopropyl steht; und $R_3$ Wasserstoff oder eine OH-Gruppe bedeutet;
wobei, wenn $R_3$ Wasserstoff ist, eine 22, 23-Doppelbindung oder -Einfachbindung vorliegt und, wenn $R_3$ eine OH-Gruppe bedeutet, eine 22,23-Einfachbindung vorliegt, beschrieben, sowie ihre Herstellung ausgehend von den entsprechend substituierten 15-Ester bzw. 13β-Ester von Antibiotika S541.

EP 0 253 378 A2

0253378

CIBA-GEIGY AG
Basel (Schweiz)

5-16005/+

# 13β-Alkyl-derivate von S541-Antibiotika zur Bekämpfung von Parasiten an Nutztieren und Pflanzen

Die vorliegende Erfindung betrifft neue, verbesserte 13β-Alkyl-Derivate von Antibiotika S541 der nachstehenden Formel I, deren Herstellung sowie deren Verwendung zur Bekämpfung von Schädlingen wie Ekto- und Endoparasiten an Tieren und Pflanzenparasiten.

Bei den erfindungsgemässen Verbindungen handelt es sich um 13β-Alkyl-Derivate von Antibiotika S541 der allgemeinen Formel I

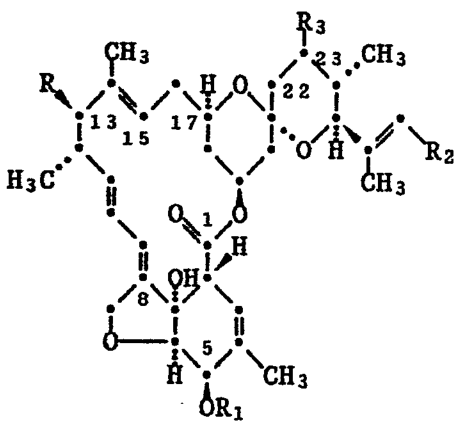

(I),

worin

R für $C_1$-$C_{10}$-Alkyl steht;

$R_1$ Wasserstoff, eine Methyl-, eine Acyl- oder eine Silylgruppe darstellt;

$R_2$ für Methyl, Ethyl oder Isopropyl steht; und
$R_3$ Wasserstoff oder eine OH-Gruppe bedeutet; wobei, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt.

Formel I repräsentiert somit Antibiotika S541-Abkömmlinge, die eine 13β-Alkylgruppe enthalten und in 5-Position entweder eine freie OH-Gruppe, eine Methoxygruppe oder eine Silyloxygruppe enthalten, sowie deren 23-Deoxy- bzw. 23-Deoxy-22,23-dehydro-Analoga.

Unter dem Begriff Alkyl selbst oder als Bestandteil eines anderen Substituenten sind je nach Zahl der angegebenen Kohlenstoffatome beispielsweise folgende geradkettige oder verzweigte Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw..

Halogen steht für Fluor, Chlor, Brom und Jod, vorzugsweise für Fluor, Chlor oder Brom. Die Vorsilbe Halo besagt, dass der entsprechende Substituent durch ein oder mehrere Halogenatome aus der Reihe Fluor, Chlor, Brom und Jod substituiert sein kann.

Bevorzugte Acylgruppen für den Substituenten $R_1$ sind z.B. die Gruppen

a) $-C(O)-\underset{R_{10}}{\underset{|}{CH}}-X-R_{11}$,

b) $-C(O)-\underset{R_{10}}{\underset{|}{CH}}-X-C(O)-R_{11}$

und

c) $-C(O)-\underset{R_{10}}{\underset{|}{CH}}-R_{11}$ ,

worin X für Sauerstoff oder Schwefel steht;

$R_{10}$ für Wasserstoff, $C_1$-$C_4$-Alkyl oder Halogen steht; und

$R_{11}$ Wasserstoff, $C_1$-$C_{10}$-Alkyl, durch Halogen, Hydroxy, $C_1$-$C_6$-Alkoxy oder $C_1$-$C_6$-Haloalkoxy substituiertes $C_1$-$C_{10}$-Alkyl, Phenyl oder durch Halogen, Cyano, $C_1$-$C_3$-Alkyl, $C_1$-$C_3$-Alkoxy, $C_1$-$C_3$-Haloalkoxy und/oder Nitro substituiertes Phenyl bedeutet.

Besonders bevorzugte Acylgruppen a), b) und c) sind solche, worin X für Sauerstoff steht;

$R_{10}$ für Wasserstoff, Methyl, Fluor, Chlor oder Brom steht und

$R_{11}$ Wasserstoff, $C_1$-$C_4$-Alkyl, durch Fluor, Chlor, Brom, Hydroxy, $C_1$-$C_4$-Alkoxy oder $C_1$-$C_4$-Haloalkoxy substituiertes $C_1$-$C_4$-Alkyl, Phenyl oder durch Fluor, Chlor, Brom, Methyl, Methoxy oder Trifluormethyl substituiertes Phenyl bedeutet.

Als geeignete Silylgruppen für $R_1$ kommt der Rest $-Si(R_4)(R_5)(R_6)$ in Frage, wobei $R_4$, $R_5$ und $R_6$ vorzugsweise unabhängig voneinander für $C_1$-$C_4$-Alkyl, Benzyl oder Phenyl stehen und beispielsweise eine der Gruppen Trimethylsilyl, tris(tert.Butyl)silyl, Dimethyl-(2,3-dimethyl-2-butyl)-silyl, Diphenyl-tert.butylsilyl, bis(Isopropyl)-methylsilyl, Triphenylsilyl usw. und insbesondere tert.Butyl-dimethylsilyl bildet. Die 5-OH-Gruppe kann auch verethert als Methylether, Benzylether oder Methoxiethoximethylether vorliegen.

Verbindungen der Formel I, worin $R_1$ eine Silylgruppe darstellt, lassen sich durch einfache, z.B. hydrolytische Abspaltung dieser Funktionen in die hochaktiven freien 5-Hydroxi-derivate ($R_1$=H) überführen und haben somit eher Zwischenprodukte-Charakter. Im übrigen wird der biologische Wert dieser Verbindungen durch die Schutzgruppe im Prinzip nicht gemindert.

Die Substituenten R in 13-Position bedeuten in natürlich vorkommenden Antibiotika S541 ($R_1$=H oder $CH_3$; $R_2$=$CH_3$, $C_2H_5$ oder iso$C_3H_7$; $R_3$=OH) stets Wasserstoff.

Eine wichtige Gruppe im Rahmen der Formel I bilden jene Verbindungen, worin

R für $C_1$-$C_{10}$-Alkyl steht;

$R_1$ Wasserstoff, eine Methyl- oder eine Silylgruppe darstellt;

$R_2$ für Methyl, Ethyl oder Isopropyl steht; und

$R_3$ Wasserstoff oder eine OH-Gruppe bedeutet;
wobei, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt.

Die natürlichen Antibiotika S541 sind aus der DE-35 32 794 und der DE-35 19 834 bekannt. Ihre Konstitution sieht wie folgt aus:

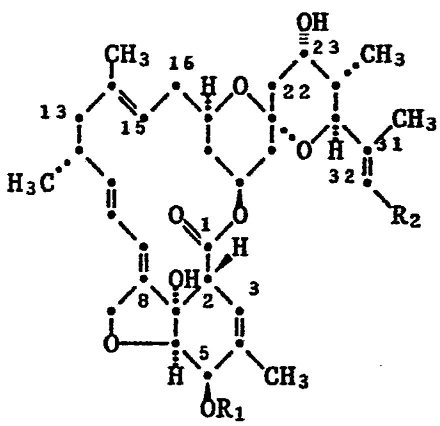

(Antibiotika S541)

| | | |
|---|---|---|
| Faktor A | $R_2$=iso$C_3H_7$ | $R_1$=H |
| Faktor B | $R_2$=$CH_3$ | $R_1$=$CH_3$ |
| Faktor C | $R_2$=$CH_3$ | $R_1$=H |
| Faktor D | $R_2$=$C_2H_5$ | $R_1$=H |
| Faktor E | $R_2$=$C_2H_5$ | $R_1$=$CH_3$ |
| Faktor F | $R_2$=iso$C_3H_7$ | $R_1$=$CH_3$ |

Je nach Faktor A, B, C, D, E oder F werden im folgenden zur Vereinfachung der Benennung die Abkömmlinge von Antibiotikum S541 als Derivate von S541A, S541B, S541C, S541D, S541E oder S541F klassifiziert.

Folgende Untergruppen von Verbindungen der Formel I sind auf Grund ihrer ausgeprägten parasitiziden und insektiziden Wirkung bevorzugt; wobei insbesondere diejenigen bevorzugt sind, worin R für $C_1$-$C_3$-Alkyl und $R_1$ für Wasserstoff steht:

Gruppe Ia: Verbindungen der Formel I, worin R für $C_1$-$C_{10}$-Alkyl steht; $R_1$ H, $CH_3$ oder die Gruppe $-Si(R_4)(R_5)(R_6)$ repräsentiert, wobei $R_4$, $R_5$ und $R_6$ unabhängig voneinander für $C_1$-$C_4$-Alkyl, Benzyl oder Phenyl stehen; $R_2$ für Methyl, Ethyl oder Isopropyl steht; und $R_3$ Wasserstoff oder eine OH-Gruppe bedeutet; wobei, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt.

Gruppe Ib: Diejenigen Verbindungen innerhalb der Untergruppe Ia, worin R für $C_1$-$C_4$-Alkyl steht; $R_1$ Wasserstoff, $CH_3$, Trimethylsilyl, tris(tert.-Butyl)silyl, Dimethyl-(2,3-dimethyl-2-butyl)-silyl, Diphenyl-tert.-butylsilyl, bis(Isopropyl)methylsilyl, Triphenylsilyl oder tert.-Butyl-dimethylsilyl bedeutet; $R_2$ für Methyl, Ethyl oder Isopropyl steht; und $R_3$ für eine OH-Gruppe steht.

Gruppe Ic: Verbindungen der Formel I, worin R für $C_1$-$C_{10}$-Alkyl steht; $R_1$ Wasserstoff repräsentiert; $R_2$ für Methyl, Ethyl oder Isopropyl steht; und $R_3$ eine OH-Gruppe bedeutet.

Gruppe Id: Verbindungen der Formel I, worin R für $C_1$-$C_{10}$-Alkyl steht; $R_1$ Wasserstoff bedeutet; und $R_2$ für Methyl, Ethyl oder Isopropyl steht; und $R_3$ für Wasserstoff steht, wobei eine 22,23-Doppel- oder -Einfachbindung vorliegt und, wenn $R_3$ eine OH-Gruppe bedeutet, eine 22,23-Einfachbindung vorliegt. Diese sind besonders bevorzugt.

Gruppe Ie: Verbindungen der Formel I, worin R für $C_1$-$C_6$-Alkyl steht; $R_1$ Wasserstoff bedeutet; $R_2$ für Methyl, Ethyl oder Isopropyl steht; und $R_3$ Wasserstoff repräsentiert; wobei eine 22,23-Doppel- oder -Einfachbindung vorliegt und, wenn $R_3$ eine OH-Gruppe bedeutet, eine 22,23-Einfachbindung vorliegt.

Gruppe If: Verbindungen der Formel I, worin R für $C_1$-$C_6$-Alkyl steht; $R_1$ Wasserstoff bedeutet; $R_2$ für Methyl, Ethyl oder Isopropyl steht; und $R_3$ Wasserstoff repräsentiert, wobei eine 22,23-Doppelbindung vorliegt.

Gruppe Ig: Verbindungen der Formel I, worin R für Methyl, Ethyl oder n-Propyl steht; $R_1$ Wasserstoff oder Methyl bedeutet; $R_2$ für Methyl, Ethyl oder Isopropyl steht; und $R_3$ Wasserstoff bedeutet, wobei eine 22,23-Doppelbindung vorliegt.

Gruppe Ih: Verbindungen der Formel I wie in Untergruppe Ig, wobei jedoch $R_3$ Wasserstoff bedeutet und eine 22,23-Einfachbindung vorliegt.

Besonders bevorzugte Einzelsubstanzen der Formel I sind z.B.:
13β-Methyl-Antibiotikum S541A
13β-Methyl-Antibiotikum S541B
13β-n-Propyl-Antibiotikum S541A
13β-Ethyl-Antibiotikum S541A
13β-Ethyl-Antibiotikum S541B
13β-n-Propyl-Antibiotikum S541B
13β-Ethyl-Antibiotikum S541C
13β-Methyl-Antibiotikum S541C
13β-Ethyl-Antibiotikum S541D
13β-Methyl-Antibiotikum S541D
13β-Ethyl-Antibiotikum S541E
13β-Methyl-Antibiotikum S541E
13β-Ethyl-Antibiotikum S541F
13β-Methyl-Antibiotikum S541F
13β-Methyl-23-deoxy-Antibiotikum S541A

13β-Ethyl-23-deoxy-Antibiotikum S541A
13β-Methyl-23-deoxy-Antibiotikum S541F
13β-Ethyl-23-deoxy-Antibiotikum S541F
13β-Methyl-22,23-dehydro-23-deoxy-Antibiotikum S541A
13β-Ethyl-22,23-dehydro-23-deoxy-Antibiotikum S541F.

Besonders bevorzugt sind ferner:

13β-Methyl-5-Acetoxy-Antibiotikum S541A
13β-Methyl-5-Acetoxy-Antibiotikum S541B
13β-Methyl-5-Acetoxy-Antibiotikum S541C
13β-Methyl-5-Acetoxy-Antibiotikum S541D
13β-Methyl-5-Acetoxy-Antibiotikum S541E
13β-Methyl-5-Acetoxy-Antibiotikum S541F.

Die vorliegende Erfindung betrifft nicht nur die Verbindungen der Formel I, sondern gleichermassen das neue Verfahren zu deren Herstellung. Es wurde nämlich gefunden, dass man durch Reaktion mit Trialkylaluminium-Verbindungen der Formel $Al(R)_3$ die nachstehend definierten Allylester der Formel II, worin die allylische $OR_7$-Gruppe sich in der 15-Position des Moleküls befindet, in die Verbindungen der Formel I überführen kann, so dass der einzuführende Substituent R stereospezifisch die 13β-Position des Moleküls einnimmt und nur in untergeordnetem Masse Nebenprodukte liefert, die in 15-Position substituiert sind und die sich mit Hilfe chromatographischer Methoden von den gewünschten Endprodukten abtrennen lassen. Als $R_7$ kommen Acyl-Gruppen in Frage, so z.B.: Formyl, Acetyl, Benzoyl, Ethoxicarbonyl oder $P(=O)(OAlkyl)_2$ wie $P(=O)(OEt)_2$, Alkylsulfonyl-Reste, vorzugsweise Niederalkylsulfonyl, insbesondere Mesyl und in gewissen Fällen auch Tetrahydropyranyl, aber auch deren höhere Homologe.

Ferner wurde gefunden, dass sich auch aus Verbindungen der Formel II, die eine 13β-$OR_7$-Gruppe enthalten, unter Beibehaltung der 13β-Orientierung die Verbindungen der Formel I gewinnen lassen. Das erfindungsgemässe Verfahren bildet somit die Möglichkeit, in

der 13β-Position von Antibiotika S541-Derivaten eine Alkyl-Gruppe R gezielt einzuführen und damit zu hochwirksamen neuen Parasitiziden und Insektiziden der Formel I zu gelangen, die gleichzeitig auch für weitere Derivatisierungen verwendet werden können und gegenüber dem Grundkörper deutliche Vorteile aufweisen.

Ein weiterer Gegenstand der vorliegenden Erfindung ist somit ein Verfahren zur Herstellung der Verbindungen der Formel I, das darin besteht, dass man einen Allylester der Formel II

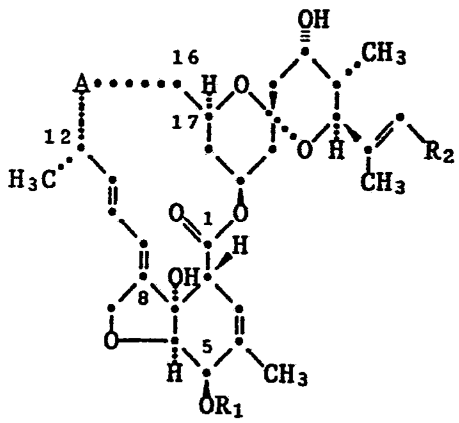

(II),

worin A für eine der Gruppen a oder b

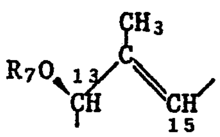

(a) oder

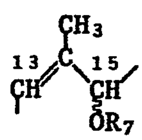

(b)

[= 13β-Ester-$\Delta^{14,15}$] [= $\Delta^{13,14}$-15-Ester]

steht, $R_7$ eine Acylgruppe repräsentiert, $R_1$ und $R_2$ die unter Formel I angegebene Bedeutung haben, mit einer Trialkylaluminium-Verbindung der Formel III

$$Al(R)_3 \qquad (III)$$

behandelt, wobei R die unter Formel I angegebenen Bedeutungen hat, woraufhin man die $R_1$-Silylgruppe, sofern freie 5-Hydroxi-Verbindungen erwünscht sind, hydrolytisch abspaltet.

Das Verfahren wird im allgemeinen in einem reaktions-inerten Lösungsmittel durchgeführt. Geeignete Lösungsmittel sind z.B.: Ether und etherartige Verbindungen wie Dialkylether (Diethylether, Diisopropylether, tert.-Butylmethylether, Dimethoxiethan, Dioxan, Tetrahydrofuran, Anisol, usw.); halogenierte Kohlenwasserstoffe wie Chlorbenzol, Methylenchlorid, Ethylenchlorid, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. Es kann von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Gewünschtenfalls können Zwischenprodukte aus dem Reaktionsmedium isoliert und falls erwünscht, vor der Weiterreaktion, auf übliche Art und Weise gereinigt werden, z.B. durch Waschen, Digerieren, Extraktion, Umkristallisation, Chromatographie usw. Mann kann jedoch auch auf derartige Reinigungsschritte verzichten und diese erst mit entsprechenden Endprodukten durchführen.

Zur Einführung der 13β-Alkylgruppe geeigneten Trialkylaluminium-Verbindungen sind $(C_1-C_{10}\text{-Alkyl})_3$Aluminium-Verbindungen, wie z.B. Trimethylaluminium, Triethylaluminium, Triisobutylaluminium, Trihexylaluminium usw. Die Reaktion wird im allgemeinen im Temperaturbereich -100°C bis 100°C, bevorzugt bei -20°C bis +60°C durchgeführt. Die Trialkyl-Aluminiumverbindung der Formel III wird dabei in Substanz oder in einem reaktionsinerten Lösungsmittel wie z.B. Hexan, Toluol oder Benzol in mindestens equimolarer Menge zur Lösung der Verbindung der Formel II gegeben.

Nach erfolgter Reaktion wird die Silyl-Schutzgruppe zweckmässigerweise durch Behandlung der Verbindungen der Formel I mit einer verdünnten Säure wie z.B. mit 1 proz. p-Toluolsulfonsäure in Methanol oder mit einer wässrigen HF-Lösung in Acetonitril im Temperaturbereich -20°C bis 50°C, bevorzugt bei 0°C bis 30°C oder mit Pyridiniumfluorid in Pyridin wieder abgespalten.

Die Trialkylaluminium-Verbindungen der Formel III sind allgemein bekannt oder lassen sich analog zu den bekannten Vertretern herstellen.

Die als Ausgangssubstanzen eingesetzten Ester der Formel II können aus den zugrundeliegenden Allylalkoholen der Formel IV

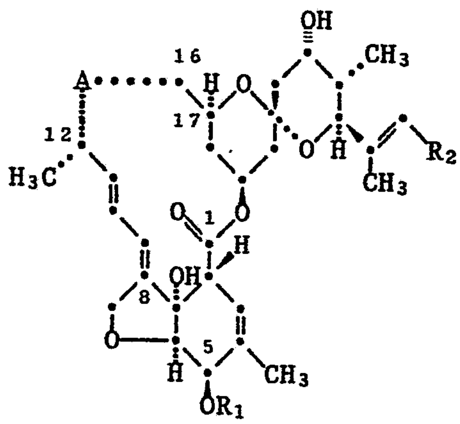

(IV),

worin A für eine der Gruppen a oder b

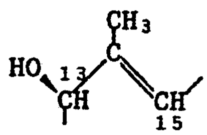

(a) oder

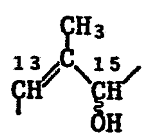

(b)

[= 13ß-Hydroxi-$\Delta^{14,15}$] [= $\Delta^{13,14}$-15-Hydroxi]

steht $R_2$ die unter Formel I angegebenen Bedeutungen hat und $R_1$ für Wasserstoff oder eine unter Formel I genannte Methyl-, Acyl- oder Silylgruppe steht; durch übliche, literaturbekannte Acylierungsmethoden wie z.B. durch Reaktionen mit Säurechloriden ($R_7COCl$) oder Säureanhydriden ($(R_7CO)_2O$), wobei $R_7$ die unter Formel II angegebenen Bedeutungen hat, in Gegenwart einer Base (Triethylamin, Pyridin, N,N-Dimethylaminopyridin usw.) in einem eingangs genannten inerten Lösungsmittel z.B. Dichlormethan, Chloroform, usw. im Temperaturbereich von -20°C bis 100°C, bevorzugt bei 0°C bis 30°C hergestellt werden.

Die Verbindungen der Formel IVb [= $\Delta^{13,14}$-15-Hydroxi] lassen sich aus 14,15-Epoxi-Verbindungen der Formel V gewinnen, worin $R_1$ und $R_2$ die für die Formel I genannten Bedeutungen haben,

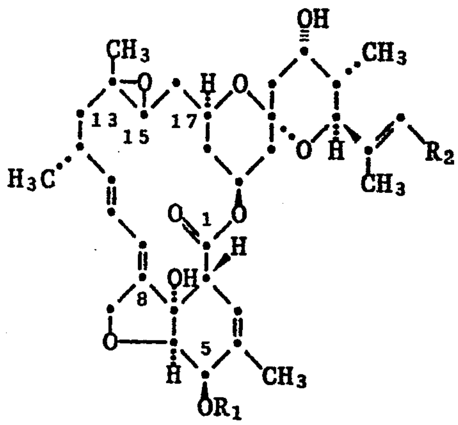

(V)

mit Hilfe des Komplex-Reagenzes $[HN_3]_x/Al(Ethyl)_3]_y$, worin x und y unabhängig voneinander die Zahl 1 oder 2 oder einen Zahlenwert zwischen 1 und 2 darstellen, in inerten trockenen Lösungsmitteln im Temperaturbereich von -20° bis +150°C, vorzugsweise +20° bis +80°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_x/[Al(Et)_3]_y$-Komplex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur

Reaktion vorgesehene Epoxi-Verbindung kann gleichfalls bereits vorliegen oder zu einem geeigneten Zeitpunkt zur Lösung dosiert werden.

Die zur Herstellung der Verbindungen IVb verwendeten Ausgangsverbindungen der Formel V lassen sich leicht durch Epoxidierung der aus der DE 35 32 794 bekanntgewordenen und als "Antibiotika S541 (Faktor A-F)" der nachstehenden Formel VI herstellen:

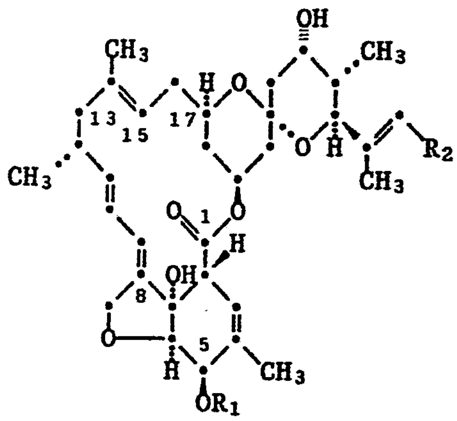

(VI)

| | | |
|---|---|---|
| Faktor A | $R_2$=iso$C_3H_7$ | $R_1$=H |
| Faktor B | $R_2$=$CH_3$ | $R_1$=$CH_3$ |
| Faktor C | $R_2$=$CH_3$ | $R_1$=H |
| Faktor D | $R_2$=$C_2H_5$ | $R_1$=H |
| Faktor E | $R_2$=$C_2H_5$ | $R_1$=$CH_3$ |
| Faktor F | $R_2$=iso$C_3H_7$ | $R_1$=$CH_3$ |

Die Epoxidierung wird in einer Lösungsmittelphase im Temperaturbereich von -10° bis +20°C, vorzugsweise -5° bis +5°C, durchgeführt.

Die Epoxidierung wird mit Persäuren wie Peressigsäure, Trifluorperessigsäure, Perbenzoesäure, Chlorperbenzoesäure durchgeführt.
Di- bzw. Triepoxide (als Nebenprodukte) können chromatographisch abgetrennt werden.

Die 13ß-Hydroxi-$\Delta^{14,15}$-Verbindungen der Formel IVa lassen sich aus Verbindungen der Formel IIb, worin $R_1$ für eine Schutzgruppe steht, durch Reaktion mit Pyridiniumdichromat [= $(Pyr)_2Cr_2O_7$] herstellen. Man arbeitet hierbei in Dimethylformamid und bei Temperaturen zwischen ca. -10° und +60°C. Die $R_1$-Schutzgruppe wird, sofern gewünscht, anschliessend hydrolytisch abgespalten.

Durch selektive Acylierung oder Silylierung der 5-OH-Gruppe werden alle jene Derivate der Formeln I bis VI hergestellt, bei denen $R_1$ eine andere Bedeutung als Wasserstoff oder $CH_3$ ($R_1$ = OH-Schutzgruppe) hat. Zur Silylierung verwendet man zweckmässigerweise ein Silan der Formel Y-Si($R_4$)($R_5$)($R_6$), worin $R_4$, $R_5$ und $R_6$ einen der eingangs genannten Reste darstellen, und wobei Y eine Silylabgangsgruppe bedeutet. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]undec-7-en (DBU) in Frage.

Die Entfernung dieser Silylreste $R_1$ in der 5-Position geschieht durch selektive milde Hydrolyse ($\longrightarrow R_1$=H) mit z.B. Arylsulfonsäure in alkoholischer Lösung oder nach einer anderen dem Fachmann geläufigen Methode.

Die Acylierung ($R_1$ = Acyl) erfolgt zweckmässigerweise an einer der Verbindungen der Formeln I bis VI, worin $R_1$ für Wasserstoff steht mit einem entsprechenden Säurehalogenid, insbesondere Säurechlorid und wird z.B. unter analogen Bedingungen wie die Silylierung durchgeführt.

Die 13β-Alkyl-Derivate von Antibiotika S541 der Formel I, worin R, $R_1$ und $R_2$ die oben angegebene Bedeutung haben und $R_3$ für eine OH-Gruppe steht, lassen sich analog zu der in US-PS 4,328,335 bekanntgewordenen Methode in die entsprechenden 23-Deoxi- bzw. 22,23-Dehydro-23-deoxy-Derivate der Formel I überführen. Dabei müssen diejenigen Verbindungen der Formel I mit einer freien 5-OH-Gruppe ($R_1$=H) zunächst selektiv geschützt werden durch Reaktion mit einem der oben angegebenen Silylierungsreagenzien $Y-Si(R_4)(R_5)(R_6)$ bzw. mit tert.-Butyldimethylsilyloxiacetylchlorid. Die Umsetzung dieser geschützten Verbindungen der Formel I mit $R_1=Si(R_4)(R_5)(R_6)$ bzw. $C(=O)CH_2OSi(CH_3)_2t-C_4H_9$ und $R_3$=OH mit p-Methylphenyl-chlorthionoformiat ergibt Derivate der Formel I in welchen $R_3$ für $O-C(=S)-OC_6H_4-CH_3-p$ steht. Diese 23-O-(4-Methyl-phenoxi)-thiocarbonyl-derivate von Antibiotika S541 werden dann als Ausgangsmaterialien für die Reduktion mit Tributylzinnhydrid in Toluol in Gegenwart von Azobisisobutyronitril bei 80-120°C zu den 23-Deoxi-Derivaten der Formel I ($R_1$=H) bzw. für die Thermolyse (150-250°C) zu den 22,23-Dehydro-23-deoxi-Derivaten der Formel I ($R_1$=H, 22,23-Doppelbindung) eingesetzt.

Die Herstellung von Verbindungen der Formel I, worin $R_3$ eine OH-Gruppe repräsentiert, kann auch erfindungsgemäss nach folgendem Reaktionsschema vorgenommen werden:

Antibiotikum S541A

$MnO_2$ — Oxydation der 5-OH-Gruppe zur 5 Ketogruppe

5-Keto-Derivat der Formel XV

1) $SeO_2/HCOOH$ 2) H+ — Einführung der 13-OH-Gruppe

13-Hydroxy-5-keto-Derivat der Formel XII

$ClCOOC_2H_5$ — Einführung der 13-$OCOOC_2H_5$-Gruppe

13-Ethoxycarbonyloxy-5-keto-Derivat der Formel XIV

$NaBH_4$ — Reduktion der 5-Ketogruppe zu 5-OH

13-Ethoxycarbonyloxy-5-hydroxy-Derivat der Formel X

$(Alkyl)_3Al$ — Einführung des Alkylrestes in die 3-Position

13-Alkyl-antibiotikum S541A der Formel I, worin $R_3$ für die OH-Gruppe steht.

Die Herstellung von Verbindungen der Formel I, worin $R_3$ eine OH-Gruppe repräsentiert, wird demnach erfindungsgemäss auch so durchgeführt, dass man ein Antibiotikum S541A-Derivat der Formel X

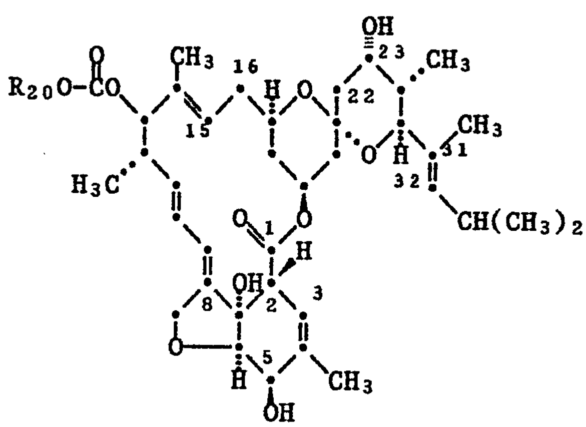

(X),

worin $R_{20}$ für $C_1$-$C_4$-Alkyl steht, mit einer Verbindung der Formel XI

$$(R)_3Al \qquad (XI),$$

worin R die unter Formel I abgegebenen Bedeutungen hat, umsetzt.

Die Reaktion von Verbindungen der Formel X mit Verbindungen der Formel XI wird normalerweise unter Inertgasatmosphäre, wie z.B. unter Stickstoff oder Argon und in Gegenwart mindestens eines wasserfreien Lösungsmittels durchgeführt. Solche Lösungsmittel schliessen z.B. halogenierte Kohlenwasserstoffe, wie Methylenchlorid, Chloroform und Dichlorethan; aromatische Kohlenwasserstoffe, wie Benzol und Toluol; aliphatische Kohlenwasserstoffe, wie n-Hexan oder n-Heptan; und Ether, wie Tetrahydrofuran und Dimethoxyethan ein.

Normalerweise wird die Reaktion so durchgeführt, dass man das Trialkylaluminium (XI) bei niederen Temperaturen langsam zutropfen lässt und anschliessend bei langsam ansteigender Temperatur im Bereich von -78°C bis 50°C, vorzugsweise -50°C bis 25°C, über einen Zeitraum von 0,5 bis 24 Stunden, üblicherweise 2 bis 4-Stunden, zu Ende führt.

Das Ausgangsmaterial der Formel X kann dadurch hergestellt werden, dass man ein 13-Hydroxy-5-keto-antibiotikum-S541A-derivat der Formel XII

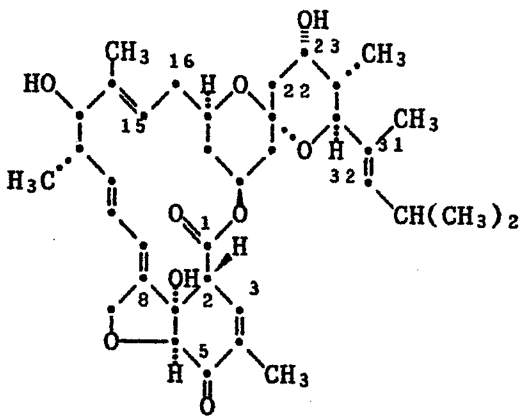

(XII)

mit einem Carbamat der Formel XIII

$R_{20}$ OCOOH                                                    (XIII)

oder einem seiner reaktionsfähigen Derivate, worin $R_{20}$ wie oben definiert ist, zu einem 13-Alkoxycarbonyloxy-5-keto-antibiotikum-S541A-derivat der Formel XIV

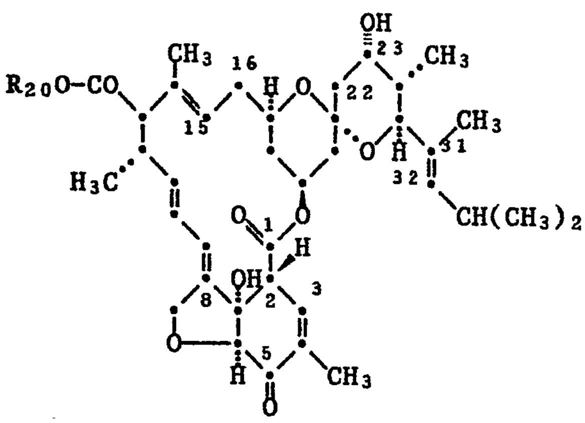

(XIV),

worin $R_{20}$ wie weiter oben definiert ist, reagieren lässt und anschliessend die Verbindung der Formel XIV zur Verbindung der Formel X reduziert.

Die Reaktion der Verbindungen der Formel XII mit Carbonaten der Formel XIII stellt eine übliche Veresterung dar, die nach an sich bekannten Methoden durchgeführt werden kann. Die reaktiven Derivate von Verbindungen der Formel XIII schliessen z.B. die entsprechenden Säurechloride, Säureanhydride, die gemischten Säureanhydride und aktiven Ester ein, von denen das Ethylchloroformiat bevorzugt ist.

Die Umsetzung erfolgt normalerweise in einem inerten Lösungsmittel und in Gegenwart eines säurebindenden Agens. Bevorzugte Lösungsmittel schliessen beispielsweise halogenierte Kohlenwasserstoffe, wie Tetrachlorkohlenstoff, Chloroform, Methylenchlorid und Dichlorethan; aromatische Kohlenwasserstoffe, wie Benzol und Toluol; und Ether, wie Diethylether, Tetrahydrofuran und Dimethoxyethan ein.

Bevorzugte säurebindende Agentien schliessen z.B. organische Amine, wie Pyridin, 4-Dimethylaminopyridin, Triethylamin, Tri-n-butylamin und tert.-Octylamin ein.

Normalerweise wird die Reaktion bei Temperaturen im Bereich von -20°C bis 80°C, vorzugsweise 0°C bos 20°C und über einen Zeitraum von 10 Minuten bis 24 Stunden, üblicherweise 30 Minuten bis 3 Stunden, durchgeführt.

Die Reduktion von Verbindungen der Formel XIV zu Verbindungen der Formel X kann im Prinzip mit jedem an sich bekannten Reduktionsverfahren durchgeführt werden. Es ist jedoch notwendig, dass man darauf achtet, dass ausser der 5-Position, in der die Reduktion stattfindet, kein anderer Teil des Moleküls zerstört wird, so dass folglich der Reduktion mit anionischem Wasserstoff der Vorzug zu geben ist. Reduktionsmittel, die freigesetzten anionischen Wasserstoff freisetzen, schliessen z.B. Natriumborhydrid und Diborane ein.

Die Reduktion wird normalerweise in einem inerten Lösungsmittel, wie z.B. Methanol, Ethanol, Diethylether, Tetrahydrofuran oder Benzol, bei Temperaturen im Bereich von 0°C bis 20°C und im Zeitraum von 10 Minuten bis zu 3 Stunden durchgeführt.

Die Alkylgruppe $R_{20}$ schliesst z.B. Methyl, Ethyl, Propyl und Butyl ein, und ist vorzugsweise Ethyl.

Die 13-Hydroxy-5-keto-antibiotika S541A der Formel XII lassen sich aus dem zugrundeliegenden 5-Keto-antibiotikum S541A der Formel XV

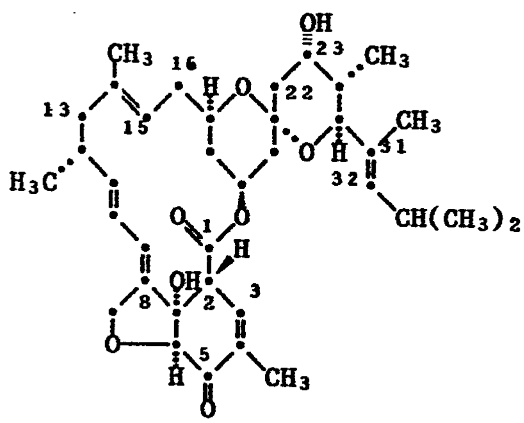

(XV)

herstellen, indem man die Verbindungen der Formel XV in Gegenwart einer Säure der Formel XVI

$$R_{21} - COOH \qquad (XVI),$$

worin $R_{21}$ Wasserstoff oder $C_1$-$C_4$-Alkyl bedeutet, mit Selendioxid reagieren lässt.

Diese Reaktion stellt eine Oxidation in der Allyl-typ-Konfiguration der 13-Position des Antibiotikums S541A dar und wird durchgeführt, indem man die Verbindung der Formel XV mit Selendioxid in Gegenwart der Säure XVI umsetzt. In den Fällen, in denen $R_{21}$ in XVI eine $C_1$-$C_4$-Alkylgruppe darstellt, kann es sich um jedes der geradkettigen oder verzweigten $C_1$-$C_4$-Alkyle handeln. Bevorzugte Säuren der Formel XVI sind Ameisensäure und Essigsäure, insbesondere jedoch die Ameisensäure. Man verwendet vorzugsweise mindestens ein Mol der Säure XVI auf ein Mol der 5-Ketoverbindung der Formel XV, insbesondere mehr als ein Mol der Säure auf ein Mol der 5-Ketoverbindung.

Die Säure kann in signifikantem Ueberschuss eingesetzt werden und gleichzeitig als Reaktionsmedium dienen, und letzteres ist in der Tat bevorzugt.

Wird statt der Säure als Lösungsmittel eine andere Substanz eingesetzt, so ist die Natur dieses Solvens nicht kritisch, vorausgesetzt, dass sie die Reaktion nicht nachteilig beeinflusst. Beispiele derartiger Lösungsmittel schliessen z.B. Kohlenwasserstoffe, vorzugsweise aliphatische und aromatische Kohlenwasserstoffe, wie Hexan oder Benzol; halogenierte Kohlenwasserstoffe, vorzugsweise aliphatische halogenierte Kohlenwasserstoffe, wie Methylenchlorid oder Chloroform; Ether, wie Diethylether, Tetrahydrofuran oder Dioxan; Alkohole, wie Methanol oder Ethanol; Ester, wie Ethylacetat oder Phenylacetat; Amide, wie Dimethylformamid oder Dimethylacetamid; Dimethylsulfoxid; Wasser; und Gemische zweier oder mehrere dieser Lösungsmittel untereinander, ein.

Die Menge an eingesetztem Selendioxid beträgt vorzugsweise von 1 Mol bis 10 Mole, insbesondere 1 bis 3 Mole, bezogen auf 1 Mol der 5-Ketoverbindung der Formel XV.

Die Reaktionstemperaturen sind keinen besonderen Beschränkungen unterworfen, da die Reaktion in weiten Temperaturbereichen stattfinden kann. Zweckmässigerweise wird die Reaktion bei Temperaturen von 0°C bis 80°C, bevorzugt bei Raumtemperaturen oder unter leichter Erwärmung durchgeführt. Bei derartigen Temperaturen liegt die Reaktionsdauer normalerweise im Bereich von 30 Minuten bis zu einem Tag.

Die 5-Ketoverbindungen der Formel XV können auf einfache Weise durch Oxidation, z.B. mit Mangandioxid oder anderen üblichen Oxidationsmitteln mittels konventioneller Oxidationsmethoden aus den Antibiotika S541A hergestellt werden.

Die Ausgangsprodukte der Formeln XI, XIII und XVI sind bekannt oder lassen sich analog zu ihren bekannten Vertretern herstellen.

Die Zwischenprodukte der Formeln X, XII, XIV und XV sind aufgrund ihrer spezifischen Struktur für die Herstellung der überaus aktiven Substanzen der Formel I prädestiniert. Sie zeigen ebenfalls parasitizide Wirkung und bilden einen Bestandteil der vorliegenden Erfindung.

Die beschriebenen Verfahren zur Herstellung der Verbindungen der Formel I sind in allen Teilschritten ein Bestandteil vorliegender Erfindung.

Ein weiterer Gegenstand vorliegender Erfindung betrifft Schädlingsbekämpfungsmittel gegen Ekto- und Endoparasiten sowie Schadinsekten, die neben üblichen Trägerstoffen und/oder Verteilungsmitteln als mindestens einen Wirkstoff eine Verbindung der Formel I enthalten.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxiuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Herstellung von Ausgangs- und Zwischenprodukten

Sofern nicht speziell erwähnt, werden bei den nachfolgenden Herstellungsmethoden die Reaktanden in äquimolaren Mengen zur Reaktion gebracht. Im allgemeinen wird der makrocyclische Reaktionspartner vorgelegt und der weitere Reaktand in äquimolarer Menge oder in leichtem Ueberschuss portionsweise zugegeben.

Beispiel A1: Herstellung von 14,15-Epoxi-Antibiotikum S541A

Zu einer Lösung von Antibiotikum S541A (Faktor A), in Dichlormethan wird unter Eiskühlung eine Lösung von m-Chlorperbenzoesäure in Dichlorethan zugegeben. Nach einstündigem Rühren bei Raumtemperatur wird zwischen Dichlormethan und 5%iger wässriger $NaHCO_3$-Lösung extrahiert. Die Chromatograhie des Rohprodukts an Kieselgel liefert 14,15-Epoxi-Antibiotikum S541A.

Beispiel A2: Herstellung von 5-O-tert.-Butyldimethylsilyl-14,15-epoxi-antibiotikum S541A

Eine Lösung von 14,15-Epoxi-Antibiotikum S541A, tert.-Butyldimethylchlorsilan und Imidazol in Dimethylformamid (DMF) wird 2 Stunden bei Raumtemperatur gerührt. Anschliessend wird Diethylether zugegeben und das Gemisch wird über Kieselgel filtriert. Nach dem Einengen wird 5-O-tert.-Butyldimethylsilyl-14,15-epoxi-antibiotikum S541A erhalten, das säulenchromatographisch gereinigt wird.

Beispiel A3: Herstellung von 5-O-tert.-Butyldimethylsilyl-15-hydroxi-$\Delta^{13,14}$-antibiotikum S541A

Eine Lösung von $HN_3/Et_3Al$-Komplex-Reagenz (hergestellt aus $Et_3Al$ und $HN_3$ in Tetrahydrofuran) wird unter Argon zu einer Lösung von 5-O-tert.-Butyldimethylsilyl-14,15-epoxi-antibiotikum S541A in Tetrahydrofuran gegeben und das Gemisch wird 12 Stunden unter Rückfluss erhitzt. Anschliessend wird bei Raumtemperatur Diethylether, Methanol und $Na_2SO_4 \cdot 10H_2O$ zugegeben.

Das Gemisch wird filtriert, eingeengt und die Chromatographie des Rohprodukts an Kieselgel ergibt 5-O-tert.-Butyldimethylsilyl-15-hydroxi-$\Delta^{13,14}$-antibiotikum S541A.

Beispiel A4: Herstellung von 5-O-tert.-Butyldimethylsilyl-15-acetoxi-$\Delta^{13,14}$-antibiotikum S541A

Eine Lösung von 5-O-tert.-Butyldimethylsilyl-15-hydroxi-$\Delta^{13,14}$-antibiotikum S541A in Essigsäureanhydrid und Pyridin wird 0,5 Stunden bei Raumtemperatur gerührt. Die Aufarbeitung in Diethylether mit

5%iger wässriger $NaHCO_3$-Lösung und einmolarer HCl und Filtration durch Kieselgel ergibt 5-O-tert.-Butyldimethylsilyl-15-acetoxi-$\Delta^{13,14}$-antibiotikum S541A.

Herstellung der Endprodukte

Beispiel H1: Herstellung von 5-O-tert.-Butyldimethylsilyl-13-methyl-antibiotikum S541A

Zu einer Lösung von 5-O-tert.-Butyldimethylsilyl-15-acetoxi-$\Delta^{13,14}$-antibiotikum S541A in Dichlormethan wird unter Argon bei 0°C eine 17%ige Lösung von Trimethylaluminium in Toluol unter Rühren zugetropft. Die Lösung wird 2 Stunden bei Raumtemperatur gerührt, dann wird mit Diethylether verdünnt und mit Celite gerührt. Die Filtration durch Kieselgel ergibt 5-O-tert.-Butyldimethylsilyl-13β-methyl-antibiotikum S541A.

Beispiel H2: a) Herstellung von 13β-Methyl-antibiotikum S541A

Eine Lösung von 5-O-tert.-Butyldimethylsilyl-13β-methyl-antibiotikum S541A in Dichlormethan wird zusammen mit einer 40%igen wässrigen Lösung von HF in Acetonitril (5:95) 1 Stunde bei Raumtemperatur gerührt. Das Gemisch wird in Diethylether aufgenommen, mit verdünnter, wässeriger $NaHCO_3$-Lösung und Wasser gewaschen, mit $MgSO_4$ getrocknet und durch Kieselgel filtriert. Die Chromatographie an Kieselgel ergibt 13β-Methyl-antibiotikum S541A.

$^1H$-NMR (270 MHz; $CDCl_3$; TMS):
3.75 (d, 1H, H in 25-Position; J = 10.9 Hz)
3.95 (d, 1H, H in 6-Position; J = 6.4 Hz)
4.28 (breites s, 1H, H in 5-Position)
5.05 (dd, 1H, H in 15-Position; J = 10.0 Hz, 5 Hz)

Massenspektrum (m/e): 626 ($M^+$), 608, 590, 480, 151.

b) Herstellung von 13β-Ethyl-antibiotikum S541A

Analog zur Herstellung der 13β-Methyl-Verbindung des Beispiels H2a erhält man die 13β-Ethyl-Verbindung.

$^1$H-NMR (270 MHz; $CDCl_3$; TMS):
3.59 (d, 1H, OH in 23-Position; J = 10.1 Hz)
3.75 (d, 1H, H in 25-Position; J = 10.9 Hz)
3.95 (d, 1H, H in 6-Position; J = 6.4 Hz)
4.29 (t, 1H, H in 5-Position; J = 6.4 Hz)
5.03 (dd, 1H, H in 15-Position; J = 11.3 Hz, 4.4 Hz)

Massenspektrum (m/e): 640 ($M^+$), 622, 604, 586, 494, 382, 342, 151.

Beispiel H3: Herstellung von 5-O-tert.-Butyldimethylsilyl-13β-methyl-23-O-(4-methylphenoxi)-thiocarbonyl-antibiotikum S541A

p-Methylphenylchlorthionoformiat wird zu einer Lösung von 5-O-tert.-Butyldimethylsilyl-13β-methylantibiotikum S541A in Pyridin bei 0°C unter Argon zugegeben. Nach 5-stündigem Rühren bei Raumtemperatur wird das Gemisch in Diethylether aufgenommen, mit Wasser gewaschen und mit $MgSO_4$ getrocknet. Die Chromatographie an Kieselgel liefert 5-O-tert.-Butyldimethylsilyl-13β-methyl-23-O-(4-methylphenoxi)thiocarbonyl-antibiotikum S541A.

Beispiel H4: Herstellung von 5-O-tert.-Butyldimethylsilyl-13β-methyl-23-deoxi-antibiotikum S541A

Eine Lösung von Tributylzinnhydrid in Toluol wird zu einer Lösung von 5-O-tert.-Butyldimethylsilyl-13β-methyl-23-O-(4'-methylphenoxi)-thiocarbonyl-antibiotikum S541A und Azobisisobutyronitril in Toluol unter Argon bei 120°C zugegeben. Nach 60 Minuten wird das Lösungsmittel abgedampft und das Rohprodukt an Kieselgel chromatographiert. Man erhält 5-O-tert.-Butyldimethylsilyl-13β-methyl-23-deoxi-antibiotikum S541A.

Beispiel H5: Herstellung von 13β-Methyl-23-deoxi-antibiotikum S541A

Eine Lösung von 5-O-tert.-Butyldimethylsilyl-13β-methyl-23-deoxi-antibiotikum S541A in Dichlormethan wird zusammen mit einer 40%igen wässrigen Lösung von HF in Acetonitril (5:95) 1 Stunde bei Raumtemperatur gerührt. Das Gemisch wird wie in Beispiel H2 in Diethylether aufgearbeitet und durch Kieselgel filtriert. Die Chromatographie an Kieselgel ergibt 13β-Methyl-antibiotikum S541A.

Beispiel H6: Herstellung von 5-O-tert.-Butyldimethylsilyl-13β-methyl-22,23-dehydro-23-deoxi-antibiotikum S541A

Eine Lösung von 5-O-tert.-Butyldimethylsilyl-13β-methyl-23-O-(4'-methylphenoxi)thiocarbonyl-antibiotikum S541A in 1,2,4-Trichlorbenzol wird unter Argon während 2 Stunden auf 200°C erhitzt. Die Chromatographie des Rohprodukts an Kieselgel ergibt 5-O-tert.-Butyldimethylsilyl-22,23-dehydro-23-deoxi-antibiotikum S541A.

Beispiel H7: Herstellung von 13β-Methyl-22,23-dehydro-23-deoxi-antibiotikum S541A

Eine Lösung von 5-O-tert.-Butyldimethylsilyl-13β-methyl-$\Delta^{22,23}$-23-deoxi-antibiotikum S541A in Dichlormethan wird zusammen mit einer 40%igen wässrigen Lösung von HF in Acetonitril (5:95) 1 Stunde bei Raumtemperatur gerührt. Das Gemisch wird wie in Beispiel H2 in Diethylether aufgearbeitet und durch Kieselgel filtriert. Die Chromatographie an Kieselgel ergibt 13β-Methyl-22,23-dehydro-23-deoxi-antibiotikum S541A.

Beispiel H8: Herstellung von 13β-Methyl-5-acetoxy-anti biotikum S541A

Eine Lösung von 13β-Methyl-antibiotikum S541A in Pyridin/Methylenchlorid (1:1) wird auf 0°C abgekühlt und anschliessend tropfenweise innerhalb von 2 Stunden mit Acetylchlorid versetzt. Nach weiterem zweistündigen Rühren bei 0°C werden die Lösungsmittel und überschüssiges Acetylchlorid im Vakuum entfernt und der ölige Rückstand säulenchromatographisch an Kieselgel gereinigt. Man erhält 13β-Methyl-5-acetoxy-antibiotikum S541A.

$^1H$-NMR (270 MHz; $CDCl_3$; TMS):
3.59 (d, 1H, OH in 23-Position; J = 10.1 Hz)
3.74 (d, 1H, H in 25-Position; J = 10.9 Hz)
4.05 (d, 1H, H in 6-Position; J = 5.6 Hz)
5.05 (dd, 1H, H in 15-Position; J = 11.3 Hz, 4.0 Hz)

Massenspektrum (m/e): 682 ($M^+$), 664, 590, 572, 480, 151, 95.

Beispiel H9: Herstellung von 5-Keto-Antibiotikum S541A
0,64 g aktiviertes Mangan werden einer Lösung von 61,2 mg des Antibiotikums S541A in 5 ml Aceton zugefügt. Dann wird das Gemisch bei Raumtemperatur heftig 30 Minuten lang gerührt. Das Gemisch wird anschliessend über Celite gefiltert und das Filtrat durch Evaporation eingeengt, wobei 59,3 mg der Titelsubstanz als Rohprodukt erhalten werden.

$^1H$-NMR (270 MHz; $CDCl_3$; TMS):
3.78 (s, 1H, OH in 7-Position)
3.84 (s, 1H, H in 6-Position)

Massenspektrum (m/e): 610, 592, 574.

Beispiel H10: Herstellung von 13-Hydroxy-5-keto-antibiotikum S541A
Das gemäss H9 hergestellte Rohprodukt wird gesamthaft in 3 ml Ameisensäure gelöst und mit 13 mg Selendioxid versetzt. Dann wird das Gemisch 1,5 Stunden bei Raumtemperatur gerührt und zur Entfernung des Selendioxids über Celite filtriert. Das Filtrat wird in Wasser gegossen und mit Ethylacetat extrahiert. Der getrocknete Extrakt wird durch Evaporation eingeengt und der Rückstand mit 2 ml Methanol, 3 ml Dioxan und 1 ml 2-normaler Salzsäure gemischt und das Gemisch über Nacht bei Raumtemperatur gerührt. Dieses Reaktionsgemisch wird wiederum in Wasser gegossen und mit Ethylacetat extrahiert. Der Extrakt wird über Magnesiumsulfat getrocknet und durch Evaporation eingeengt. Der Rückstand wird durch präparative Dünnschichtchromatographie (Merck Art 5715, 20 cm x 20 cm, Dicke

2 mm) gereinigt, unter Verwendung von Hexan/Ethylacetat (1:1) als Eluierungsmittel. Man erhält 13,2 mg (21,7 % Ausbeute) der Titelsubstanz.

$^1$H-NMR (270 MHz; $CDCl_3$; $D_2O$; TMS):
3.73 (d, 1H, H in 13-Position, J = 9.7 Hz)
3.75 (d, 1H, H in 25-Position, J = 10.5 Hz)
3.85 (s, 1H, H in 6-Position)

Massenspektrum (m/e): 626 ($M^+$ -36), 608, 590, 349, 331, 259, 242, 179.

Beispiel H11: Herstellung von 13-Ethoxycarbonyloxy-5-keto-antibiotikum S541A

Zu einer Lösung von 76 mg 13-Hydroxy-5-keto-antibiotikum S541A in einem Gemisch von Methylenchlorid und Tetrahydrofuran (2 ml + 1 ml) werden 58 µl Pyridin und 35 µl Ethylchloroformiat unter Eiskühlung zugegeben. Das Gemisch wird noch 4 Stunden bei Raumtemperatur gerührt, in Eiswasser gegossen und mit Ethylacetat extrahiert. Der Extrakt wird wechselweise mit Wasser und einer gesättigten, wässrigen Natriumchloridlösung gewaschen und über Magnesiumsulfat getrocknet. Das Lösungsmittel wird sodann abdestilliert, und man erhält 93 mg der Titelsubstanz als Rohprodukt.

Beispiel H12: Herstellung von 13-Ethoxycarbonyloxy-antibiotikum S541A

Das gesamte nach H11 erhaltene Rohprodukt wird in 2 ml Methanol gelöst und diese Mischung unter Eiskühlung mit 2,5 mg Natriumborhydrid versetzt. Das Reaktionsgemisch wird bei gleicher Temperatur noch 15 Minuten gerührt, in Eiswasser gegossen und mit Ethylacetat extrahiert. Der Extrakt wird abwechselnd mit Wasser und einer gesättigten, wässrigen Natriumchloridlösung gewaschen und über Natriumsulfat getrocknet. Das Lösungsmittel wird abdestilliert und der Rückstand säulenchromatographisch gereinigt (Silicagel). Man erhält 55 mg (65 % Ausbeute) der Titelsubstanz.

$^1$H-NMR (270 MHz; $CDCl_3$; TMS):
3.48 (d, 1H, OH in 23-Position, J = 10.1 Hz)
3.72 (d, 1H, H in 25-Position, J = 10.9 Hz)
3.96 (d, 1H, H in 6-Position, J = 6.0 Hz)
4.19 (m, 2H, Me-$\underline{CH_2}$-OCOO-)
4.32 (m, 1H, H in 5-Position)
4.73 (d, 1H, H in 3-Position, J = 10.5 Hz)

Massenspektrum (m/e): 700 ($M^+$), 682, 592, 554, 151, 95.

Beispiel H13: Herstellung von 13-Methyl-antibiotikum S541A

Zu einer Lösung von 30 mg von 13-Ethoxycarbonyloxy-antibiotikum S541A in 0,5 ml Methylenchlorid wird unter Eiskühlung eine Lösung von 5,7 ml 19 %igem Trimethylaluminium in Hexan zugegeben und das Gemisch bei Raumtemperatur 4 Stunden gerührt. Anschliessend wird das Gemisch in Eiswasser gegossen und nach Zusatz von 10 ml Ethylacetat über Celite filtriert. Das Celite wird in 50 ml Ethylacetat gewaschen und nach Trennung der Phasen, die wässrige Phase mit Ethylacetat extrahiert. Filtrat und Waschacetat werden vereinigt, abwechselnd mit Wasser und gesättigter, wässriger Natriumchloridlösung gewaschen und über Natriumsulfat getrocknet. Das Lösungsmittel wird entfernt und der Rückstand säulenchromatographisch über Silicagel gereinigt. Man erhält 17 mg (63 % Ausbeute) der Titelsubstanz.

$^1$H-NMR (270 MHz; $CDCl_3$; TMS):
3.75 (d, 1H, H in 25-Position; J = 10.9 Hz)
3.95 (d, 1H, H in 6-Position; J = 6.4 Hz)
4.28 (breites s, 1H, H in 5-Position)
5.05 (dd, 1H, H in 15-Position; J = 10.0 Hz, 5 Hz)

Massenspektrum (m/e): 626 ($M^+$), 608, 590, 480, 151.

0253378

Beispiel H14: Herstellung von 13-Ethyl-antibiotikum S541A
Zu einer Lösung von 20 mg von 13-Ethoxycarbonyloxy-antibiotikum S541A in 0,5 ml Methylenchlorid werden unter Eiskühlung 0,8 ml 15 %iges Triethylaluminium in Hexan zugefügt und das Reaktionsgemisch 4,5 Stunden bei Raumtemperatur gerührt. Die Aufarbeitung erfolgte in derselben Weise wie in H13 beschrieben. Man erhält 4,6 mg (25 % Ausbeute) der Titelsubstanz und als Nebenprodukt 1,4 mg (7,7 % Ausbeute) 15-Ethyl-$\Delta^{13,14}$-antibiotikum S541A.

13-Ethylverbindung:

$^1$H-NMR (270 MHz; $CDCl_3$; TMS):
3.59 (d, 1H, OH in 23-Position; J = 10.1 Hz)
3.75 (d, 1H, H in 25-Position; J = 10.9 Hz)
3.95 (d, 1H, H in 6-Position; J = 6.4 Hz)
4.29 (t, 1H, H in 5-Position; J = 6.4 Hz)
5.03 (dd, 1H, H in 15-Position; J = 11.3 Hz, 4.4 Hz)

Massenspektrum (m/e): 640 ($M^+$), 622, 604, 586, 494, 382, 342, 151.

15-Ethylverbindung:

$^1$H-NMR (270 MHz; $CDCl_3$; TMS):
3.58 (d, 1H, OH in 23-Position, J = 9.7 Hz)
4.02 (d, 1H, H in 6-Position, J = 6.5 Hz)
4.92 (d, 1H, H in 13-Position, J = 9.7 Hz)

Massenspektrum (m/e): 640 ($M^+$), 622, 604, 494, 476, 382.

Analog zu den beschriebenen Arbeitsweisen werden auch die nachfolgend genannten Verbindungen der Formel I hergestellt; wobei die nachfolgende Tabelle keinen limitierenden Charakter hat:

Tabelle 1: Typische Vertreter von Verbindungen der Formel I, worin $R_1$ für Wasserstoff steht, und $R_3$ eine OH-Gruppe bedeutet.

| Verb. Nr. | $R_2$ | R |
|---|---|---|
| 1.1 | $CH_3$ | $CH_3$ |
| 1.2 | $CH_3$ | $C_2H_5$ |
| 1.3 | $CH_3$ | $C_3H_7$-n |
| 1.4 | $CH_3$ | $C_3H_7$-iso |
| 1.5 | $CH_3$ | $C_4H_9$-n |
| 1.6 | $CH_3$ | $C_4H_9$-sek |
| 1.7 | $CH_3$ | $C_4H_9$-iso |
| 1.8 | $CH_3$ | $C_5H_{11}$-n |
| 1.9 | $CH_3$ | $C_6H_{13}$-n |
| 1.10 | $CH_3$ | $C_7H_{15}$-n |
| 1.11 | $CH_3$ | $C_8H_{17}$-n |
| 1.12 | $CH_3$ | $C_9H_{18}$-n |
| 1.13 | $CH_3$ | $C_{10}H_{21}$-n |
| 1.14 | $C_2H_5$ | $CH_3$ |
| 1.15 | $C_2H_5$ | $C_2H_5$ |
| 1.16 | $C_2H_5$ | $C_3H_7$-iso |
| 1.17 | $C_2H_5$ | $C_4H_9$-n |
| 1.18 | $C_2H_5$ | $C_4H_9$-sek |
| 1.19 | $C_2H_5$ | $C_4H_9$-iso |
| 1.20 | $C_2H_5$ | $C_5H_{11}$-n |
| 1.21 | $C_2H_5$ | $C_6H_{13}$-n |
| 1.22 | $C_3H_7$-iso | $CH_3$ |
| 1.23 | $C_3H_7$-iso | $C_2H_5$ |
| 1.24 | $C_3H_7$-iso | $C_3H_7$-n |
| 1.25 | $C_3H_7$-iso | $C_3H_7$-iso |
| 1.26 | $C_3H_7$-iso | $C_4H_9$-n |
| 1.27 | $C_3H_7$-iso | $C_4H_9$-sek |
| 1.28 | $C_3H_7$-iso | $C_4H_9$-iso |
| 1.29 | $C_3H_7$-iso | $C_6H_{13}$-n |
| 1.30 | $C_3H_7$-iso | $C_5H_{11}$-n |
| 1.31 | $C_2H_5$ | $C_3H_7$-n |

Tabelle 2: Typische Vertreter von Verbindungen der Formel I, worin $R_1$ für Wasserstoff steht, $R_3$ Wasserstoff bedeutet und eine 22,23-Doppelbindung vorliegt.

| Verb. Nr. | $R_2$ | R |
|---|---|---|
| 2.1 | $CH_3$ | $CH_3$ |
| 2.2 | $CH_3$ | $C_2H_5$ |
| 2.3 | $CH_3$ | $C_3H_7$-n |
| 2.4 | $CH_3$ | $C_3H_7$-iso |
| 2.5 | $CH_3$ | $C_4H_9$-n |
| 2.6 | $CH_3$ | $C_4H_9$-sek |
| 2.7 | $CH_3$ | $C_4H_9$-iso |
| 2.8 | $CH_3$ | $C_5H_{11}$-n |
| 2.9 | $CH_3$ | $C_6H_{13}$-n |
| 2.10 | $CH_3$ | $C_7H_{15}$-n |
| 2.11 | $CH_3$ | $C_8H_{17}$-n |
| 2.12 | $CH_3$ | $C_9H_{18}$-n |
| 2.13 | $CH_3$ | $C_{10}H_{21}$-n |
| 2.14 | $C_2H_5$ | $CH_3$ |
| 2.15 | $C_2H_5$ | $C_2H_5$ |
| 2.16 | $C_2H_5$ | $C_3H_7$-iso |
| 2.17 | $C_2H_5$ | $C_4H_9$-n |
| 2.18 | $C_2H_5$ | $C_4H_9$-sek |
| 2.19 | $C_2H_5$ | $C_4H_9$-iso |
| 2.20 | $C_2H_5$ | $C_5H_{11}$-n |
| 2.21 | $C_2H_5$ | $C_6H_{13}$-n |
| 2.22 | $C_3H_7$-iso | $CH_3$ |
| 2.23 | $C_3H_7$-iso | $C_2H_5$ |
| 2.24 | $C_3H_7$-iso | $C_3H_7$-n |
| 2.25 | $C_3H_7$-iso | $C_3H_7$-iso |
| 2.26 | $C_3H_7$-iso | $C_4H_9$-n |
| 2.27 | $C_3H_7$-iso | $C_4H_9$-sek |
| 2.28 | $C_3H_7$-iso | $C_4H_9$-iso |
| 2.29 | $C_3H_7$-iso | $C_6H_{13}$-n |
| 2.30 | $C_3H_7$-iso | $C_5H_{11}$-n |
| 2.31 | $C_2H_5$ | $C_3H_7$-n |

Tabelle 3: Typische Vertreter von Verbindungen der Formel I, worin $R_1$ für Wasserstoff steht, $R_3$ Wasserstoff bedeutet und eine 22,23-Einfachbindung vorliegt.

| Verb. Nr. | $R_2$ | R |
|---|---|---|
| 3.1 | $CH_3$ | $CH_3$ |
| 3.2 | $CH_3$ | $C_2H_5$ |
| 3.3 | $CH_3$ | $C_3H_7$-n |
| 3.4 | $CH_3$ | $C_3H_7$-iso |
| 3.5 | $CH_3$ | $C_4H_9$-n |
| 3.6 | $CH_3$ | $C_4H_9$-sek |
| 3.7 | $CH_3$ | $C_4H_9$-iso |
| 3.8 | $CH_3$ | $C_5H_{11}$-n |
| 3.9 | $CH_3$ | $C_6H_{13}$-n |
| 3.10 | $CH_3$ | $C_7H_{15}$-n |
| 3.11 | $CH_3$ | $C_8H_{17}$-n |
| 3.12 | $CH_3$ | $C_9H_{18}$-n |
| 3.13 | $CH_3$ | $C_{10}H_{21}$-n |
| 3.14 | $C_2H_5$ | $CH_3$ |
| 3.15 | $C_2H_5$ | $C_2H_5$ |
| 3.16 | $C_2H_5$ | $C_3H_7$-iso |
| 3.17 | $C_2H_5$ | $C_4H_9$-n |
| 3.18 | $C_2H_5$ | $C_4H_9$-sek |
| 3.19 | $C_2H_5$ | $C_4H_9$-iso |
| 3.20 | $C_2H_5$ | $C_5H_{11}$-n |
| 3.21 | $C_2H_5$ | $C_6H_{13}$-n |
| 3.22 | $C_3H_7$-iso | $CH_3$ |
| 3.23 | $C_3H_7$-iso | $C_2H_5$ |
| 3.24 | $C_3H_7$-iso | $C_3H_7$-n |
| 3.25 | $C_3H_7$-iso | $C_3H_7$-iso |
| 3.26 | $C_3H_7$-iso | $C_4H_9$-n |
| 3.27 | $C_3H_7$-iso | $C_4H_9$-sek |
| 3.28 | $C_3H_7$-iso | $C_4H_9$-iso |
| 3.29 | $C_3H_7$-iso | $C_6H_{13}$-n |
| 3.30 | $C_3H_7$-iso | $C_5H_{11}$-n |
| 3.31 | $C_2H_5$ | $C_3H_7$-n |

Tabelle 4: Typische Vertreter von Verbindungen der Formel I, worin $R_1$ eine Silylgruppe darstellt und $R_3$ eine OH-Gruppe bedeutet.

| Verb. Nr. | $R_2$ | R | $R_1$ |
|---|---|---|---|
| 4.1 | $CH_3$ | $CH_3$ | tert.-Butyldimethylsilyl |
| 4.2 | $CH_3$ | $C_2H_5$ | tert.-Butyldimethylsilyl |
| 4.3 | $CH_3$ | $C_3H_7$-n | tert.-Butyldimethylsilyl |
| 4.4 | $CH_3$ | $C_3H_7$-iso | tert.-Butyldimethylsilyl |
| 4.5 | $CH_3$ | $C_4H_9$-n | tert.-Butyldimethylsilyl |
| 4.6 | $CH_3$ | $C_4H_9$-sek | tert.-Butyldimethylsilyl |
| 4.7 | $CH_3$ | $C_4H_9$-tert | tert.-Butyldimethylsilyl |
| 4.8 | $C_2H_5$ | $CH_3$ | tert.-Butyldimethylsilyl |
| 4.9 | $C_3H_7$-iso | $CH_3$ | tert.-Butyldimethylsilyl |
| 4.10 | $C_3H_7$-iso | $CH_3$ | tert.-Butyldimethylsilyl |
| 4.11 | $C_2H_5$ | $C_2H_5$ | tert.-Butyldimethylsilyl |
| 4.12 | $C_2H_5$ | $C_3H_7$-iso | tert.-Butyldimethylsilyl |
| 4.13 | $C_3H_7$-iso | $C_2H_5$ | tert.-Butyldimethylsilyl |
| 4.14 | $CH_3$ | $CH_3$ | Trimethylsilyl |
| 4.15 | $CH_3$ | $CH_3$ | tris(tert.-Butyl)silyl |
| 4.16 | $CH_3$ | $CH_3$ | Diphenyl-tert.-butylsilyl |
| 4.17 | $CH_3$ | $CH_3$ | bis(Isopropyl)methylsilyl |
| 4.18 | $CH_3$ | $CH_3$ | Triphenylsilyl |

Tabelle 5: Typische Vertreter der Formel I, worin $R_1$ eine Acylgruppe darstellt und $R_3$ eine OH-Gruppe repräsentiert.

| Verb. Nr. | R | $R_2$ | $R_1$ |
|---|---|---|---|
| 5.1 | $CH_3$ | $CH_3$ | $COCH_3$ |
| 5.2 | $CH_3$ | $CH_3$ | $COCH_2Cl$ |
| 5.3 | $CH_3$ | $CH_3$ | $COCH_2Br$ |
| 5.4 | $CH_3$ | $CH_3$ | $COCHBrF$ |
| 5.5 | $CH_3$ | $CH_3$ | $COCH_2F$ |
| 5.6 | $CH_3$ | $CH_3$ | $COCH_2OCH_3$ |
| 5.7 | $CH_3$ | $CH_3$ | $COCH_2OSCH_3$ |
| 5.8 | $CH_3$ | $CH_3$ | $COCH_2OCOCH_3$ |
| 5.9 | $CH_3$ | $CH_3$ | $COCHFOCOCH_3$ |
| 5.10 | $CH_3$ | $CH_3$ | $COCH_2OCOC_2H_5$ |
| 5.11 | $CH_3$ | $CH_3$ | $COCH_2OCOC_6H_4F(3)$ |
| 5.12 | $CH_3$ | $CH_3$ | $COCH_2OCOC_6H_4Cl(3)$ |
| 5.13 | $CH_3$ | $CH_3$ | $COCH_2OCOC_6H_4OCH_3(3)$ |
| 5.14 | $CH_3$ | $CH_3$ | $COCH_2OCOC_6H_3Cl_2(2,4)$ |
| 5.15 | $C_2H_5$ | $CH_3$ | $COCH_3$ |
| 5.16 | $C_2H_5$ | $CH_3$ | $COCH_2OCH_3$ |
| 5.17 | $C_2H_5$ | $CH_3$ | $COCH_2OCOC_6H_5$ |
| 5.18 | $CH_3$ | $C_2H_5$ | $COCH_3$ |
| 5.19 | $CH_3$ | $C_2H_5$ | $COCH_2OCH_3$ |
| 5.20 | $CH_3$ | $C_3H_7$-iso | $COCH_3$ |
| 5.21 | $CH_3$ | $C_3H_7$-iso | $COCH_2OCH_3$ |
| 5.22 | $C_2H_5$ | $C_3H_7$-iso | $COCH_3$ |
| 5.23 | $C_2H_5$ | $C_3H_7$-iso | $COCH_2Cl$ |

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus den Tabellen | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff aus den Tabellen | 10 % |
| Na-Ligninsulfonat | 2 % |
| Carboximethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

| | | |
|---|---|---|
| I | Ein Wirkstoff aus den Tabellen | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Verbindungen der Formeln I, insbesondere solche aus Tabellen 1-3 und 4, erzielen bereits bei einer Wirkstoffkonzentration von 3 ppm eine vollständige Abtötung nach 24 Stunden.

B-2. Wirkung gegen pflanzenschädigende Akariden
OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25°C.

Nach sieben Tagen wird unter dem Binokular auf Prozentsatz mobiler Stadien (Adulte und Nymphen) und auf vorhandene Eier ausgewertet.

Verbindungen der Formel I worin $R_1$ für Wasserstoff steht und R einen Niederalkylrest darstellt erzielen bereits bei einer Wirkstoffkonzentration von 0,4 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I aus den Tabellen 1-3 und 4 erzielen mit 100 ppm eine Wirkung von 100 %.

B-4. Akarizide Wirkung gegen Boophilus microplus (Biarra-Stamm

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel

1 µl einer Flüssigkeit injiziert, die eine 1:1-Mischung von Polyethylenglykol und Aceton darstellt und in der eine bestimmte Wirkstoffmenge von wahlweise 1, 0,1 oder 0,01 µg pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I aus den Tabellen 1-3 und 4, insbesondere diejenigen, worin R niedere Alkylreste darstellt, erzielen eine $IR_{90}$ von 0,1 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I aus Tabellen 1-3 und 4, vorzugsweise mit einer Verbindung der Formel I, worin R für Niederalkyl steht, behandelt werden, zeigen im Vergleich zu unbehandelten, aber infizierten Vergleichsgruppen keinen Nematodenbefall (= komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Verbindungen der Formel I wie z.B. Vertreter aus den Tabellen 1-3 und 4 erzielen bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1%igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I, insbeonsere solche aus den Tabellen 1-3 und 4 bewirken in diesem Test bei einer Konzentration von 1,6 ppm bereits nach einem Tag eine vollständige Abtötung sämtlicher Larven.

B-8. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten

Zecken zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen zeigen bei 100 ppm eine Wirkung von 100 %.

Bei allen biologischen Tests B1 bis B8 erzielen Verbindungen der Formel I, worin R für Methyl oder Ethyl steht, $R_3$ Wasserstoff bedeutet, $R_2$ für Methyl, Ethyl oder Isopropyl steht und $R_1$ Wasserstoff bedeutet, eine hervorragende Wirkung und sind tendenziell aktiver als die entsprechenden Vertreter, worin $R_3$ für Wasserstoff steht.

0253378

Patentansprüche

1. Verbindungen der Formel I,

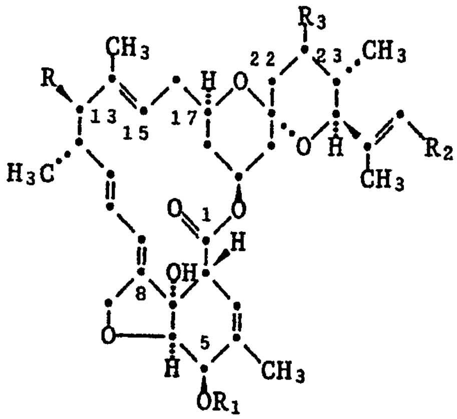

(I)

worin

R für $C_1$-$C_{10}$-Alkyl steht;

$R_1$ Wasserstoff, eine Methyl-, eine Acyl- oder eine Silylgruppe darstellt;

$R_2$ für Methyl, Ethyl oder Isopropyl steht; und

$R_3$ Wasserstoff oder eine OH-Gruppe bedeutet; wobei, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt.

2. Verbindungen der Formel I nach Anspruch 1,
worin

R für $C_1$-$C_{10}$-Alkyl steht;

$R_1$ Wasserstoff, eine Methyl- oder eine Silylgruppe darstellt;

$R_2$ für Methyl, Ethyl oder Isopropyl steht; und

$R_3$ Wasserstoff oder eine OH-Gruppe bedeutet;

wobei, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt.

3. Verbindungen der Formel I nach Anspruch 2, worin R für $C_1$-$C_{10}$-Alkyl steht; $R_1$ H, $CH_3$ oder die Gruppe -Si($R_4$)($R_5$)($R_6$) repräsentiert, wobei $R_4$, $R_5$ und $R_6$ unabhängig voneinander für $C_1$-$C_4$-Alkyl, Benzyl oder Phenyl stehen; $R_2$ für Methyl, Ethyl oder Isopropyl steht; und $R_3$ Wasserstoff oder eine OH-Gruppe bedeutet; wobei, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt.

4. Verbindungen der Formel I nach Anspruch 3, worin R für $C_1$-$C_4$-Alkyl steht; $R_1$ Wasserstoff, $CH_3$, Trimethylsilyl, tris(tert.-Butyl)-silyl, Dimethyl-(2,3-dimethyl-2-butyl)-silyl, Diphenyl-tert.-butylsilyl, bis(Isopropyl)methylsilyl, Triphenylsilyl oder tert.-Butyldimethylsilyl bedeutet; $R_2$ für Methyl, Ethyl oder Isopropyl steht; und $R_3$ für eine OH-Gruppe steht.

5. Verbindungen der Formel I nach Anspruch 2, worin R für $C_1$-$C_{10}$-Alkyl steht; $R_1$ Wasserstoff repräsentiert; $R_2$ für Methyl, Ethyl oder Isopropyl steht; und $R_3$ eine OH-Gruppe bedeutet.

6. Verbindungen der Formel I nach Anspruch 2, worin R für $C_1$-$C_{10}$-Alkyl steht; $R_1$ Wasserstoff bedeutet; und $R_2$ für Methyl, Ethyl oder Isopropyl steht; und $R_3$ Wasserstoff steht, wobei eine 22,23-Doppel- oder -Einfachbindung vorliegt.

7. Verbindungen der Formel I nach Anspruch 2, worin R für $C_1$-$C_6$-Alkyl steht; $R_1$ Wasserstoff bedeutet; $R_2$ für Methyl, Ethyl oder Isopropyl steht; und $R_3$ Wasserstoff repräsentiert; wobei eine 22,23-Doppel- oder -Einfachbindung vorliegt.

8. Verbindungen der Formel I nach Anspruch 7, worin R für $C_1$-$C_6$-Alkyl steht; $R_1$ Wasserstoff bedeutet; $R_2$ für Methyl, Ethyl oder Isopropyl steht; und $R_3$ Wasserstoff repräsentiert, wobei eine 22,23-Doppelbindung vorliegt.

9. Verbindungen der Formel I nach Anspruch 8, worin R für Methyl, Ethyl oder n-Propyl steht; $R_1$ Wasserstoff oder Methyl bedeutet; $R_2$ für Methyl, Ethyl oder Isopropyl steht; und $R_3$ Wasserstoff bedeutet, wobei eine 22,23-Doppelbindung vorliegt.

10. Verbindungen der Formel I nach Anspruch 5, worin R für Methyl, Ethyl oder n-Propyl steht; $R_1$ Wasserstoff oder Methyl bedeutet; $R_2$ für Methyl, Ethyl oder Isopropyl steht; und $R_3$ Wasserstoff bedeutet, wobei eine 22,23-Einfachbindung vorliegt.

11. Eine Verbindung der Formel I nach Anspruch 2, ausgewählt aus der Reihe:

13β-Methyl-Antibiotikum S541A
13β-Methyl-Antibiotikum S541B
13β-n-Propyl-Antibiotikum S541A
13β-Ethyl-Antibiotikum S541A
13β-Ethyl-Antibiotikum S541B
13β-n-Propyl-Antibiotikum S541B
13β-Ethyl-Antibiotikum S541C
13β-Methyl-Antibiotikum S541C
13β-Ethyl-Antibiotikum S541D
13β-Methyl-Antibiotikum S541D
13β-Ethyl-Antibiotikum S541E
13β-Methyl-Antibiotikum S541E
13β-Ethyl-Antibiotikum S541F
13β-Methyl-Antibiotikum S541F
13β-Methyl-23-deoxy-Antibiotikum S541A
13β-Ethyl-23-deoxy-Antibiotikum S541A
13β-Methyl-23-deoxy-Antibiotikum S541F
13β-Ethyl-23-deoxy-Antibiotikum S541F
13β-Methyl-22,23-dehydro-23-deoxy-Antibiotikum S541A und
13β-Ethyl-22,23-dehydro-23-deoxy-Antibiotikum S541F.

12. Eine Verbindung der Formel I nach Anspruch 1, ausgewählt aus der Reihe:

13β-Methyl-5-Acetoxy-Antibiotikum S541A
13β-Methyl-5-Acetoxy-Antibiotikum S541B
13β-Methyl-5-Acetoxy-Antibiotikum S541C
13β-Methyl-5-Acetoxy-Antibiotikum S541D
13β-Methyl-5-Acetoxy-Antibiotikum S541E und
13β-Methyl-5-Acetoxy-Antibiotikum S541F.

13. Verfahren zur Herstellung von Verbindungen der Formel I,

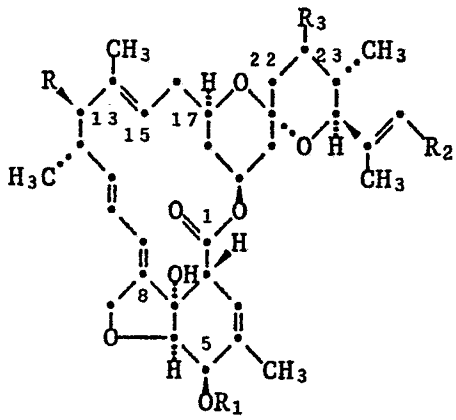

(I)

worin
R für $C_1$-$C_{10}$-Alkyl steht;
$R_1$ Wasserstoff, eine Methyl-, eine Acyl- oder eine Silylgruppe darstellt;
$R_2$ für Methyl, Ethyl oder Isopropyl steht;
$R_3$ Wasserstoff oder eine OH-Gruppe bedeutet; und, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt, dadurch gekennzeichnet, dass man einen Allylester der Formel II

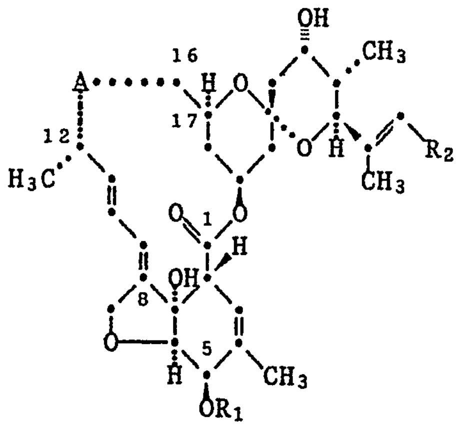

(II)

worin A für eine der Gruppen a oder b

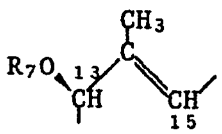

(a) oder

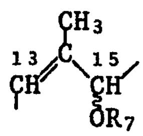

(b)

[= 13β-Ester-$\Delta^{14,15}$] [= $\Delta^{13,14}$-15-Ester]

steht, $R_7$ eine Acylgruppe repräsentiert, $R_1$ und $R_2$ die unter Formel I angegebene Bedeutung haben, mit einer Trialkylaluminium-Verbindung der Formel III

$$Al(R)_3 \qquad (III)$$

behandelt, wobei R die unter Formel I angegebenen Bedeutungen hat, woraufhin man die $R_1$-Silylgruppe, sofern freie 5-Hydroxy-Verbindungen erwünscht sind, hydrolytisch abspaltet, und gegebenenfalls die 23-Hydroxy-Gruppe reduziert bzw. eliminiert.

14. Verfahren nach Anspruch 13, dadurch gekennzeichnet, dass man die Umsetzung von II und III in einem reaktionsinerten Lösungsmittel bei Temperaturen von -100°C bis +100°C durchführt.

15. Schädlingsbekämpfungsmittel gegen Ekto-, Endoparasiten an Tieren oder Pflanzen und gegen schädliche Insekten, dadurch gekennzeichnet, dass es neben üblichen Trägerstoffen und/oder Verteilungsmitteln mindestens eine Verbindung der Formel I,

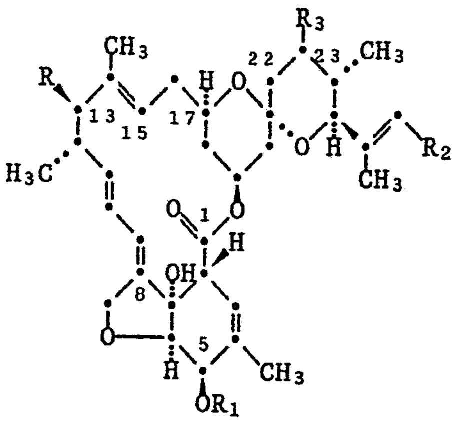

(I)

worin

R für $C_1$-$C_{10}$-Alkyl steht;

$R_1$ Wasserstoff, eine Methyl-, eine Acyl- oder eine Silylgruppe darstellt;

$R_2$ für Methyl, Ethyl oder Isopropyl steht;

$R_3$ Wasserstoff oder eine OH-Gruppe bedeutet; und, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt, enthält.

16. Mittel gemäss Anspruch 15, dadurch gekennzeichnet, dass es als Verbindung der Formel I mindestens eine Verbindung gemäss den Ansprüchen 2 bis 12 enthält.

17. Verfahren zur Bekämpfung von Schädlingen an Nutztieren und Pflanzen, dadurch gekennzeichnet, dass man eine Verbindung der Formel I,

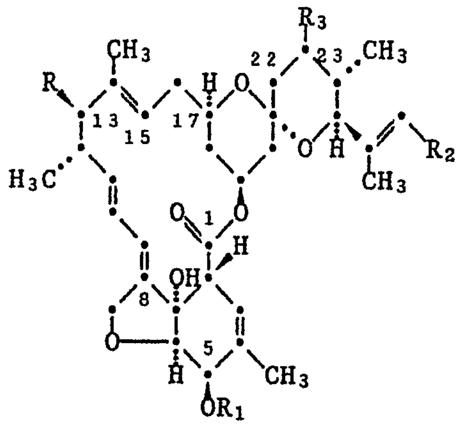

(I)

worin

R für $C_1$-$C_{10}$-Alkyl steht;

$R_1$ Wasserstoff, eine Methyl-, eine Acyl- oder eine Silylgruppe darstellt;

$R_2$ für Methyl, Ethyl oder Isopropyl steht;

$R_3$ Wasserstoff oder eine OH-Gruppe bedeutet; und, wenn $R_3$ Wasserstoff ist, eine 22,23-Doppelbindung oder -Einfachbindung vorliegt und, wenn $R_3$ eine OH-Gruppe bedeutet, eine 22,23-Einfachbindung vorliegt, enthält,

in einer parasitizid bzw. insektizid wirksamen Menge auf das Nutztier, die Pflanze oder den Lebensraum beider oder den Lebensraum des Parasiten appliziert.

18. Verfahren nach Anspruch 17, dadurch gekennzeichnet, dass es sich bei der applizierten Substanz um eine Verbindung der Formel I gemäss einem der Ansprüche 2 bis 12 handelt.

19. Verfahren nach Anspruch 17, dadurch gekennzeichnet, dass es sich bei den Schädlingen um phytopathogene Ektoparasiten und Insekten handelt.

20. Verfahren nach Anspruch 17, dadurch gekennzeichnet, dass es sich bei den Schädlingen um Endoparasiten im Warmblüter handelt.

21. Verfahren nach Anspruch 20, dadurch gekennzeichnet, dass es sich bei den Endoparasiten um Nematoden handelt.

22. Verfahren zur Herstellung von Verbindungen der Formel I

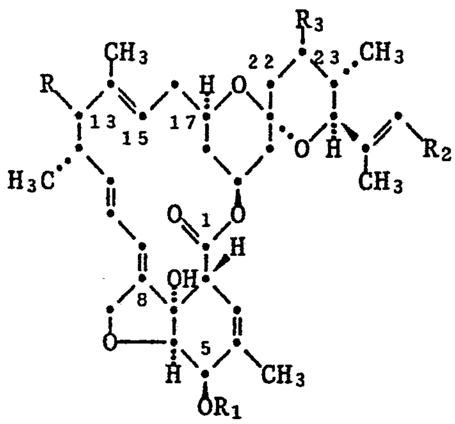

(I),

worin

R für $C_1$-$C_{10}$-Alkyl steht;

$R_1$ Wasserstoff, eine Methyl-, eine Acyl- oder eine Silylgruppe darstellt;

$R_2$ für Methyl, Ethyl oder Isopropyl steht; und

$R_3$ eine OH-Gruppe bedeutet;

dadurch gekennzeichnet, dass man ein Antibiotikum S541A-Derivat der Formel X

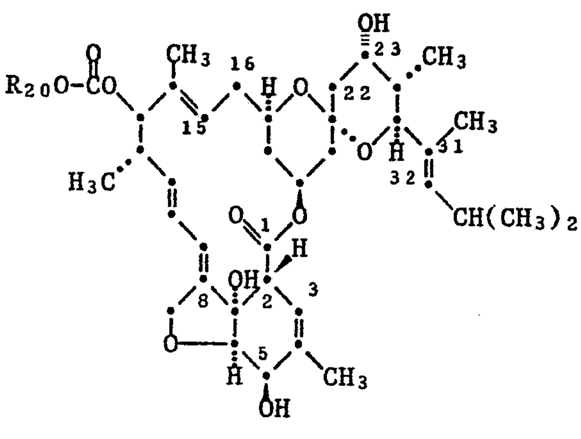

(X),

worin $R_{20}$ für $C_1$-$C_4$-Alkyl steht, mit einer Verbindung der Formel XI

$(R)_3Al$ (XI),

worin R die unter Formel I abgegebenen Bedeutungen hat, umsetzt und sofern gewünscht, an der 5-OH-Gruppe syliliert oder acyliert.

23. Verbindungen der Formel X

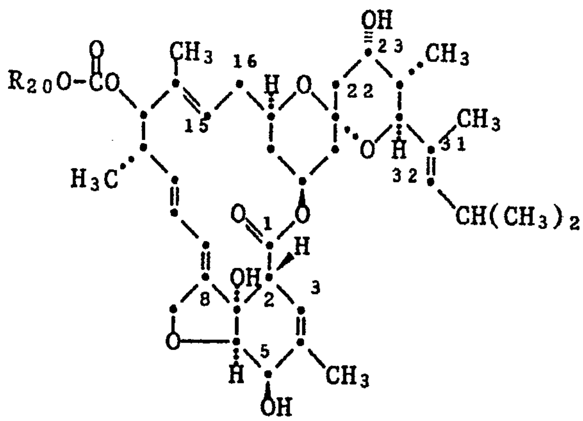

(X),

worin $R_{20}$ für $C_1$-$C_4$-Alkyl steht.

24. Verbindungen der Formel XII

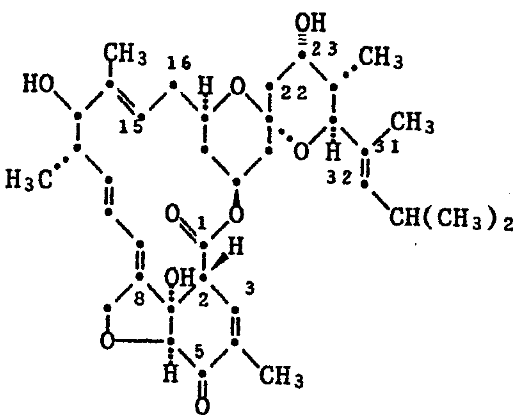

(XII).

25. Verbindungen der Formel XIV

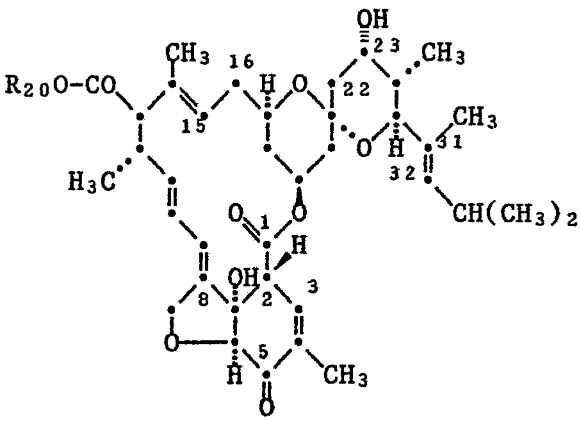

(XIV),

worin $R_{20}$ für $C_1$-$C_4$-Alkyl steht.

26. Die Verbindung der Formel XV

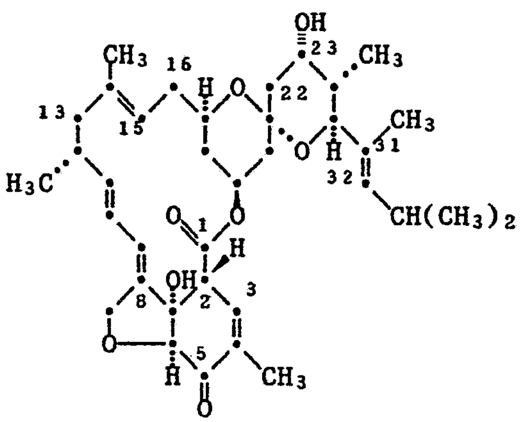

(XV).

FO 7.5 HL/gb*/hpw*